# EUROPEAN PATENT SPECIFICATION

(11) **EP 2 637 746 B1**
(45) Date of publication and mention of the grant of the patent: **30.11.2016**
(21) Application number: 11779125.1
(22) Date of filing: 21.10.2011
(51) Int. Cl.: A61Q 19/08, A61K 8/37, A61K 8/67, A61K 8/365

(54) **LEAVE-ON NON-SOLID SKIN CONDITIONING COMPOSITIONS CONTAINING 12-[(12-HYDROXYOCTADECANOYL)OXY]OCTADECANOIC ACID**
AUF DER HAUT VERBLEIBENDE NICHTFESTE HAUTPFLEGEZUSAMMENSETZUNGEN enthaltend 12-[(12-HYDROXYOCTADECANOYL)OXY]OCTADECANSÄURE
COMPOSITIONS CONDITIONANTES NON SOLIDES À LAISSER SUR LA PEAU, CONTENANT DE L'ACIDE 12-[(12-HYDROXYOCTADÉCANOYL)OXY] OCTADÉCANOÏQUE

(30) Priority: 11.11.2010 US 944289
(43) Date of publication of application: 18.09.2013
(73) Proprietor: Unilever PLC, London, Greater London EC4Y 0DY (GB); Unilever N.V., 3013 AL Rotterdam (NL)
(72) Inventor: MADISON, Stephen, Alan, Trumbull, Connecticut 06611 (US); MOADDEL, Teanoosh, Trumbull, Connecticut 06611 (US); HARICHIAN, Bijan, Trumbull, Connecticut 06611 (US); ROSA, Jose, Guillermo, Trumbull, Connecticut 06611 (US); MELDRUM, Helen, Trumbull, Connecticut 06611 (US); LEE, Jianming, Trumbull, Connecticut 06611 (US)
(74) Representative: Corsten, Michael Allan
(86) International application number: PCT/EP2011/068428
(87) International publication number: WO 2012/062554

(56) References cited:
- DE-A1- 19 756 377
- US-A- 5 552 136
- US-B1- 6 423 325

## Description

### BACKGROUND OF THE INVENTION

The desire to look young and healthy is universal. Outward signs of aging are perceived primarily through skin appearance. Signs of skin aging include wrinkles, sagging, age spots, hyperpigmentation, etc. These signs may be the result of chronological aging or photo-damage. Despite many cosmetic products marketed for this purpose, a need remains for skin leave-on products which reduce signs of aging.

Human skin consists of two major layers: dermis - the bottom thicker layer, and epidermis - the top thinner layer. Dermis is the layer that provides strength, elasticity, and thickness to the skin. With aging, the thickness and strength of the dermal layer is reduced, thus partially causing the formation of wrinkles/sagging in aging skin. Fibroblasts are the main cell type in the dermal layer. It is well known that chronological aging and photoaging lead to inadequate energy production in dermal fibroblasts, causing functional and structural alterations in skin. The top layer of the skin, the epidermis, provides the resilience and the barrier properties of the skin. The epidermis is composed of many different cell types. Keratinocytes are the major cell type of the epidermis, consisting of nearly 75 to 80 % of the total number of cells in the human epidermis. Keratinocytes reside in four distinct stages of differentiation within the epidermis. Epidermal differentiation aids the formation of a barrier layer that protects the body against the harmful substances in the environment and prevents loss of water from the body. Proper formation of the barrier layer of the epidermis requires skin cells to develop correctly through space and time.

12-hydroxystearic acid (12HSA) is reported to have a wide variety of beneficial cosmetic effects on skin, e.g. it is a known PPAR-alpha (peroxisome proliferator activated receptors sub-type alpha) activator, skin lightening agent, and a sebum secretion inhibitor. See e.g. Alaluf et al. US 6 423 325, Mayes et al. US 6 713 051, WO 2006/056283 (Hindustan Lever), Madison US 2009/0317341, Minami et al. US 6 197 343, Granger et al. US 2004/0043044. Unfortunately 12HSA is a solid and has no water solubility and limited oil solubility. Indeed 12HSA has traditionally been used as gelling agent e.g. in lipsticks and anti-perspirant compositions. See also EP 0 129 528, US 6 680 285, Abbas et al. US 6 680 285, Tanner et al. US 5 759 524, WO 95/31961 (Procter & Gamble), Kawa et al., US 2004/0044078 (describing the use of 12HSA to increase viscosity of cosmetic compositions), and JP 2010/138 110. Salts of 12HSA are only marginally more water-soluble. Commercial samples of 12HSA are only about 90 % pure. As part of the present invention, the inventors identified a specific impurity present in many commercial samples of 12HSA, which is a specific derivative of 12-hydroxystearic acid, namely 12-[(12-hydroxyoctadecanoyl)oxy] octadecanoic acid. Inventors also found that, surprisingly, 12-[(12-hydroxyoctadecanoyl)oxy] octadecanoic acid can deliver a benefit within the dermal layer of the skin, which is not attained by 12-hydroxystearic acid ("12HSA") itself. The inventors also found that 12-[(12-hydroxyoctadecanoyl)oxy] octadecanoic acid does not act as an oil gellant (as 12HSA does) but instead upon cooling of a heated sample mixtures of 12-[(12-hydroxyoctadecanoyl)oxy] octadecanoic acid with oils (specifically, numerous oils that are routinely used as carriers and/or emollients in the leave-on non-solid compositions) of interest remain as a flowable liquid. The inventors also found that in the epidermal layer 12HSA induces keratinocyte differentiation, but 12-[(12-hydroxyoctadecanoyl)oxy] octadecanoic acid does not. Surprisingly, 12-[(12-hydroxyoctadecanoyl)oxy] octadecanoic acid, does however stimulate energy production in the form of ATP by human fibroblasts, whereas 12HSA does not. Thus the present invention is based at least in part on the findings that 12-[(12-hydroxyoctadecanoyl)oxy] octadecanoic acid has unique benefits and properties desirable for skin conditioning leave-on non-solid compositions, and is especially useful in combination with 12HSA.

### SUMMARY OF THE INVENTION

The present invention includes leave-on non-solid skin conditioning composition comprising:
(a) 12-[(12-hydroxyoctadecanoyl)oxy] octadecanoic acid; and
(b) a cosmetically acceptable carrier, preferably lipohilic and liquid at temperatures up to 40°C;
wherein the non-solid skin conditioning composition is an emulsion or a cream.

Especially preferred compositions also include 12HSA.

The invention also includes methods of skin conditioning by applying to the skin the compositions according to the present invention.

### DETAILED DESCRIPTION OF THE INVENTION

Except in the examples, or where otherwise explicitly indicated, all numbers in this description indicating amounts of material or conditions of reaction, physical properties of materials and/or use are to be understood as modified by the word "about." All amounts are by weight of the final composition, unless otherwise specified.

It should be noted that in specifying any range of concentration or amount, any particular upper concentration can be associated with any particular lower concentration or amount.

For the avoidance of doubt, the word "comprising" is intended to mean "including" but not necessarily "consisting of" or "composed of." In other words, the listed steps or options need not be exhaustive.

"Leave-on" as used herein means compositions that are applied to the skin and are not intended to be washed or rinsed off for some period of time, as contrasted with cleansing or wash-off or rinse-off compositions.

"Non-solid" as used herein means that the composition has a measurable viscosity (measurable for instance with a Brookfield Viscometer DV-I + (20RPM, RV6, 30 Seconds) in the range of from 1 Pas to 500 Pas, preferably from 2Pas to 100 Pas, more preferably from 3Pas to 50Pas.

"Flowable liquid" as used herein includes mixtures that are liquid at temperatures up to about 40°C, including dispersions, emulsions and solutions.

### 12-[(12-hydroxyoctadecanoyl)oxy] octadecanoic acid

### METHOD OF MAKING

12-[(12-hydroxyoctadecanoyl)oxy] octadecanoic acid may be prepared as depicted below (Scheme I). Briefly, 12HSA is strategically derivatized at the carboxylic acid and hydroxyl functional groups with orthogonal protecting groups as illustrated by compounds (4) and (3) below. Coupling between the free carboxylic acid and hydroxyl functional groups gives the diprotected 12-[(12-hydroxyoctadecanoyl)oxy] octadecanoic acid (5). Sequential removal of the hydroxyl protecting group to give compound (6), followed by removal of the carboxylic acid protecting group gives the desired 12-[(12-hydroxyoctadecanoyl)oxy] octadecanoic acid (7).

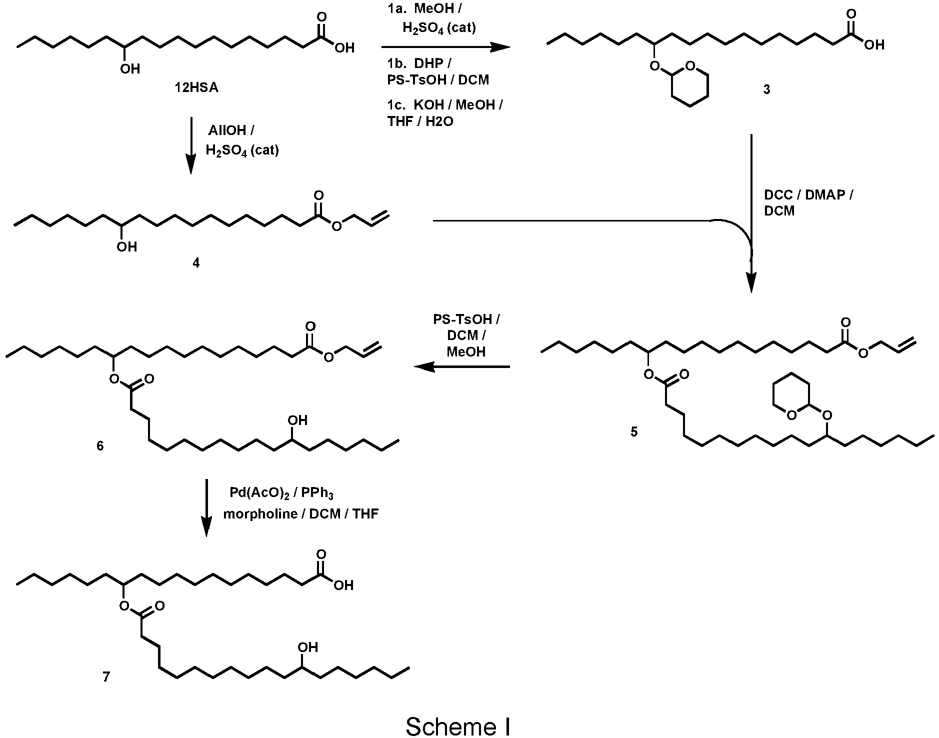

Typically, 12-[(12-hydroxyoctadecanoyl)oxy] octadecanoic acid is used in amount of up to 10 % by weight of the composition, preferably up to 5 %, most preferably up to 1 %, preferably from 0.0001 % to 3 %. It has been found that very small amounts attain increased energy production by human fibroblasts.

Mono- and divalent salts of 12-[(12-hydroxyoctadecanoyl)oxy] octadecanoic acid may be included in the amounts stated above.

### COSMETICALLY ACCEPTABLE CARRIER

Compositions of this invention will also include a cosmetically acceptable carrier. According to the present invention, lipophilic carriers which are liquids at temperatures up to 40°C are preferred since 12-[(12-hydroxyoctadecanoyl)oxy] octadecanoic acid is soluble in such carriers.

Amounts of the carrier may range from about 1 to about 99.9 %, preferably from about 70 to about 95 %, optimally from about 80 to about 90 % by weight of the composition. Among the useful carriers are water, emollients, fatty acids, fatty alcohols, thickeners and combinations thereof. The carrier may be aqueous, anhydrous or an emulsion. Preferably the compositions are aqueous, especially water and oil emulsions of the W/O or O/W type or multiple emulsions of the W/O/W or O/ W/O variety. Water when present may be in amounts ranging from about 5 to about 95 %, preferably from about 20 to about 70 %, optimally from about 35 to about 60 % by weight.

Emollient materials may serve as cosmetically acceptable carriers. These may be in the form of silicone oils, natural or synthetic esters, hydrocarbons, alcohols and fatty acids. Amounts of the emollients may range anywhere from about 0.1 to about 95 %, preferably between about 1 and about 50 % by weight of the composition.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 5 to 6, silicon atoms.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 x 10⁻⁶ to 0.1 m²/s at 25°C. Among the preferred non-volatile emollients useful in the present compositions are the polydimethyl siloxanes having viscosities from about 1 x 10⁻⁵ to about 4 x 10⁻⁴ m²/s at 25°C.

Another class of non-volatile silicones are emulsifying and non-emulsifying silicone elastomers. Representative of this category is Dimethicone/Vinyl Dimethicone Crosspolymer available as Dow Corning 9040, General Electric SFE 839, and Shin-Etsu KSG-18. Silicone waxes such as Silwax WS-L (Dimethicone Copolyol Laurate) may also be useful.

Among the ester emollients are:
a) Alkyl esters of saturated fatty acids having 10 to 24 carbon atoms. Examples thereof include behenyl neopentanoate, isononyl isonanonoate, isopropyl myristate and octyl stearate.
b) Ether-esters such as fatty acid esters of ethoxylated saturated fatty alcohols.
c) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
d) Wax esters such as beeswax, spermaceti wax and tribehenin wax.
e) Sugar ester of fatty acids such as sucrose polybehenate and sucrose polycottonseedate.

Natural ester emollients principally are based upon mono-, di- and tri- glycerides. Representative glycerides include sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof. Animal derived emollients are represented by lanolin oil and lanolin derivatives. Amounts of the natural esters may range from about 0.1 to about 20 % by weight of the compositions.

Hydrocarbons which are suitable cosmetically acceptable carriers include petrolatum, mineral oil, C₁₁-C₁₃ isoparaffins, polybutenes and especially isohexadecane, available commercially as Permethyl 101A from Presperse Inc. Fatty acids having from 10 to 30 carbon atoms may also be suitable as cosmetically acceptable carriers. Illustrative of this category are pelargonic, lauric, myristic, palmitic, stearic, isostearic, oleic, linoleic, linolenic, hydroxystearic and behenic acids and mixtures thereof.

Fatty alcohols having from 10 to 30 carbon atoms are another useful category of cosmetically acceptable carrier. Illustrative of this category are stearyl alcohol, lauryl alcohol, myristyl alcohol, oleyl alcohol and cetyl alcohol and mixtures thereof.

Thickeners can be utilized as part of the cosmetically acceptable carrier of compositions according to the present invention. Typical thickeners include crosslinked acrylates (e.g. Carbopol 982®), hydrophobically-modified acrylates (e.g. Carbopol 1382®), polyacrylamides (e.g. Sepigel 305®), acryloylmethylpropane sulfonic acid/salt polymers and copolymers (e.g. Aristoflex HMB® and AVC®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, talc, calcium carbonate and silicates such as magnesium aluminum silicate (Veegum®). Amounts of the thickener may range from 0.0001 to 10 %, usually from 0.001 to 1 %, optimally from 0.01 to 0.5 % by weight of the composition.

Preferred are emollients that can be used, especially for products intended to be applied to the face, to improve sensory properties and are chosen from the group of oils that do not form stiff gels with 12HSA; these include polypropylene glycol-14 butyl ether otherwise known as Tegosoft PBE, or PPG15 stearyl ether such as Tegosoft E, other oils such as esters, specifically, isopropyl myristate, isopropyl palmitate, other oils could include castor oils and derivatives thereof.

Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5 to 50 %, preferably between 1 and 15 % by weight of the composition.

Skin moisturizers, e.g. hyaluronic acid and/or its precursor N-acetyl glucosamine may be included. N-acetyl glucosamine may be found in shark cartilage or shitake mushrooms and are available commercially from Maypro Industries, Inc (New York). Other preferred moisturizing agents include hydroxypropyl tri(C₁-C₃ alkyl)ammonium salts. These salts may be obtained in a variety of synthetic procedures, most particularly by hydrolysis of chlorohydroxypropyl tri(C₁-C₃ alkyl)ammonium salts. A most preferred species is 1,2-dihydroxypropyltrimonium chloride, wherein the C₁-C₃ alkyl is a methyl group. Amounts of the salt may range from about 0.2 to about 30 %, and preferably from about 0.5 to about 20 %, optimally from about 1 % to about 12 % by weight of the topical composition, including all ranges subsumed therein.

Ordinarily the C₁-C₃ alkyl constituent on the quaternized ammonium group will be methyl, ethyl, n-propyl, isopropyl or hydroxyethyl and mixtures thereof. Particularly preferred is a trimethyl ammonium group known through INCI nomenclature as a "trimonium" group. Any anion can be used in the quat salt. The anion may be organic or inorganic with proviso that the material is cosmetically acceptable. Typical inorganic anions are halides, sulfates, phosphates, nitrates and borates. Most preferred are the halides, especially chloride. Organic anionic counter ions include methosulfate, toluoyl sulfate, acetate, citrate, tartrate, lactate, gluconate, and benzenesulfonate.

Still other preferred moisturizing agents which may be used, especially in conjunction with the aforementioned ammonium salts include substituted urea like hydroxymethyl urea, hydroxyethyl urea, hydroxypropyl urea; bis(hydroxymethyl) urea; bis(hydroxyethyl) urea; bis(hydroxypropyl) urea; N,N'-dihydroxymethyl urea; N,N'-di-hydroxyethyl urea; N,N'-di-hydroxypropyl urea; N,N,N'-tri-hydroxyethyl urea; tetra(hydroxymethyl) urea; tetra(hydroxyethyl) urea; tetra(hydroxypropyl urea; N-methyl, N'-hydroxyethyl urea; N-ethyl-N'-hydroxyethyl urea; N-hydroxypropyl-N'-hydroxyethyl urea and N,N'dimethyl-N-hydroxyethyl urea. Where the term hydroypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50 % aqueous liquid from the National Starch & Chemical Division of ICI under the trademark Hydrovance.

Amounts of substituted urea that may be used in the topical composition of this invention range from about 0.01 to about 20 %, and preferably, from about 0.5 to about 15 %, and most preferably, from about 2 to about 10 % based on total weight of the composition and including all ranges subsumed therein.

When ammonium salt and substituted urea are used, in a most especially preferred embodiment at least from about 0.01 to about 25 %, and preferably, from about 0.2 to about 20 %, and most preferably, from about 1 to about 15 % humectant, like glycerine, is used, based on total weight of the topical composition and including all ranges subsumed therein.

### Form of the Composition

The compositions of the present invention are non-solid. Essentially, the "non-solidness" of the composition means that the viscosity of the compositions, e.g. as measured using a Brookfield DV-I + viscometer (20RPM, RV6, 30 seconds). The viscosity is in general is in the range of from 1 Pas to 500Pas, preferably from 1 Pas to 200Pas, more preferably from 2Pas to 100Pas, most preferably from 3Pas to 50Pas.

The compositions of the invention are leave-on compositions. The compositions of the present invention are intended to be applied to remain on the skin. These leave-on compositions are to be distinguished from compositions which are applied to the skin and subsequently removed either by washing, rinsing, wiping, or the like either after or during the application of the product.. Surfactants typically used for rinse-off compositions have physico-chemical properties giving them the ability to generate foam/lather in-use with ease of rinse; they can consist of mixtures of anionic, cationic, amphoteric, and nonionic. Surfactants used in leave-on compositions on the other hand are not required to have such properties. Rather, as leave-on compositions are not intended to be rinsed-off they need to be non-irritating and therefore it would be necessary to minimize the total level of surfactant and the total level of anionic surfactant in leave-on compositions. Therefore, the compositions of the present invention contain, with respect to surfactants, predominantly nonionic surfactants. The anionic surfactants are present in an amount of at most 5 %, preferably from 0.01 to 4 %, more preferably from 0.01 to 3 %, most preferably from 0.01 to 2 % and optimally are substantially absent (less than 1 %, preferably less than 0.1 %, or even less than 0.01 %). Salts of 12HSA are not considered anionic surfactants herein. The total level of surfactant in the inventive compositions is preferably no more than 10 %, more preferably below 8 %, most preferably at most 5 %.

The compositions of the present invention are typically in the form of emulsions, which may be oil-in-water, or water-in-oil; preferably the compositions are oil-in-water emulsions. Another preferred format is a cream, furthermore preferably one which has a vanishing cream base. Vanishing cream base is one which comprises 5 to 40 % fatty acid and 0.1 to 20 % soap. In such creams, the fatty acid is preferably substantially a mixture of stearic acid and palmitic acid and the soap is preferably the potassium salt of the fatty acid mixture, although other counterions and mixtures thereof can be used. The fatty acid in vanishing cream base is often prepared using hystric acid which is substantially (generally about 90 to 95 %) a mixture of stearic acid and palmitic acid. A typical hystric acid comprises about 52-55 % palmitic acid and 45-48 % stearic acid of the total palmitic-stearic mixture. Thus, inclusion of hystric acid and its soap to prepare the vanishing cream base is within the scope of the present invention. It is particularly preferred that the composition comprises higher than 7 %, preferably higher than 10 %, more preferably higher than 12 % fatty acid.

### OPTIONAL INGREDIENTS

### 12HSA

Compositions that contain both 12-[(12-hydroxyoctadecanoyl)oxy] octadecanoic acid and 12HSA are particularly preferred and may be used to deliver a variety of skin conditioning benefits. "Conditioning" as used herein includes prevention and treatment of aged and photo-damaged skin, appearance of wrinkles, age spots, aged skin, increasing skin firmness, increasing stratum corneum flexibility, lightening skin color, controlling sebum excretion and generally increasing the quality and radiance of skin. The composition may be used to improve fibroblast metabolic activity and proliferation, skin desquamation and epidermal differentiation and improve skin appearance or general aesthetics.

The compositions preferably contain at least 40 % of the total 12HSA in its acid form, preferably at least 50 %, more preferably at least 60 % in order to improve 12HSA bioavailability, and therefore efficacy. As with other fatty acids the pKa for 12HSA is expected to be greater than 8. At the pKa, the fatty acid will exist as 50 % soap and 50 % acid. Therefore, preferably the pH of the inventive compositions is less than about 8, more preferably is in the range of from 3.5 to 8.0, most preferably is from 5 to 7.8. 12HSA is preferably included in the inventive compositions in an amount of from 0.01 to 15 %, more preferably from 0.1 to 12 %, most preferably from 0.5 to 10 %, and optimally from 1 to 5 %. The amounts of 12HSA or salts thereof are not meant to be included within the surfactants amounts herein.

### Surfactants

Total concentration of the surfactant when present may range from about 0.1 to about 90 %, preferably from about 1 to about 40 %, optimally from about 1 to about 20 % by weight of the composition, and being highly dependent upon the type of personal care product. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) and trialkylamine oxides are also suitable nonionic surfactants.

Useful amphoteric surfactants include cocoamidopropyl betaine, C₁₂-C₂₀ trialkyl betaines, sodium lauroamphoacetate, and sodium laurodiamphoacetate.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, C₈-C₂₀ sarcosinates, C₈-C₂₀ acyl lactylates, sulfoacetates and combinations thereof. In compositions containing both 12-[(12-hydroxyoctadecanoyl)oxy] octadecanoic acid and 12HSA, the preferred surfactants are high HLB nonionic sugar surfactant with an HLB of at least 7 selected from the group consisting of alkyl polyglucosides, sugar fatty acid esters, aldobionamides, polyhydroxy fatty acid amides and mixtures thereof.

### Rheology Modifier

A rheology modifier may be included and is selected from the group consisting of silica such as fumed silica or hydrophilic silicas and clays such as magnesium aluminum silicate, betonites, hectorite, laponite, and mixtures thereof. A rheology modifier is employed in an amount of from 0.01 to 2 %, preferably from 0.05 to 1 %.

### Skin Benefit Ingredients

The inventive composition preferably includes an additional skin lightening compound, to obtain optimum skin lightening performance at an optimum cost. Illustrative substances are placental extract, lactic acid, niacinamide, arbutin, kojic acid, ferulic acid, hydroquinone, resorcinol and derivatives including 4-substituted resorcinols and combinations thereof. More preferably such additional skin lightening compound is a tyrosinase inhibitor to complement the melanogenesis inhibition activity of the substituted monoamines, most preferably a compound selected from the group consisting of kojic acid, hydroquinone and 4-substituted resorcinol. Also dicarboxylic acids represented by the formula HOOC-(CxHy)-COOH where x=4 to 20 and y=6 to 40 such as azelaic acid, sebacic acid, oxalic acid, succinic acid, fumaric acid, octadecenedioic acid or their salts or a mixture thereof, most preferably fumaric acid or salt thereof, especially di-sodium salt. It has been found that combination of 12HSA with fumaric acid or salts thereof are particularly preferred, especially for skin lightening formulations. Amounts of these agents may range from about 0.1 to about 10 %, preferably from about 0.5 to about 2 % by weight of the composition. It is preferred that the skin lightening coactive according to the invention is vitamin B3 or a derivative thereof and is selected from the group consisting of niacinamide, nicotinic acid esters, non-vasodilating esters of nicotinic acid, nicotinyl amino acids, nicotinyl alcohol esters of carboxylic acids, nicotinic acid N-oxide, niacinamide N-oxide and mixtures thereof.

Sunscreen is another preferred ingredient of the inventive compositions. Particularly preferred are such materials as ethylhexyl p-methoxycinnamate (available as Parsol MCX^{®}), Avobenzene (available as Parsol 1789^{®}), octylsalicylate (available as Dermablock OS^{®}), tetraphthalylidene dicamphor sulfonic acid (available as Mexoryl SX®), benzophenone-4 and benzophenone-3 (Oxybenzone). Inorganic sunscreen actives may be employed such as microfine titanium dioxide, zinc oxide, polyethylene and various other polymers. By the term "microfine" is meant particles of average size ranging from about 10 to about 200 nm, preferably from about 20 to about 100 nm. Amounts of the sunscreen agents when present may generally range from 0.1 to 30 %, preferably from 2 to 20 %, optimally from 4 to 10 % by weight of the composition.

More preferred inventive compositions include both the additional skin lightening compound, especially tyrosinase inhibitor, and a sunscreen compound.

Another preferred ingredient of the inventive compositions is a retinoid. As used herein, "retinoid" includes all natural and/or synthetic analogs of Vitamin A or retinol-like compounds which possess the biological activity of Vitamin A in the skin as well as the geometric isomers and stereoisomers of these compounds. The retinoid is preferably retinol, retinol esters (e.g., C₂ -C₂₂ alkyl esters of retinol, including retinyl palmitate, retinyl acetate, retinyl propionate), retinal, and/or retinoic acid (including all-trans retinoic acid and/or 13-cis-retinoic acid), more preferably retinoids other than retinoic acid. These compounds are well known in the art and are commercially available from a number of sources, e.g., Sigma Chemical Company (St. Louis, Mo.), and Boerhinger Mannheim (Indianapolis, Ind.). Other retinoids which are useful herein are described in US 4 677 120 issued June 30 1987 to Parish et al.; US 4 885 311 issued Dec. 5 1989 to Parish et al.; US 5 049 584 issued Sep. 17 1991 to Purcell et al.; US 5 124 356 issued Jun. 23 1992 to Purcell et al.; and US Pat. No. Reissue 34,075 issued Sep. 22 1992 to Purcell et al. Other suitable retinoids are tocopheryl-retinoate [tocopherol ester of retinoic acid (trans- or cis-), adapalene {6-[3-(1-adamantyl)-4-methoxyphenyl]-2-naphthoic acid}, and tazarotene (ethyl 6-[2-(4,4-dimethylthiochroman-6-yl)-ethynyl]nicotinate). Preferred retinoids are retinol, retinyl palmitate, retinyl acetate, retinyl propionate, retinal and combinations thereof. The retinoid is preferably substantially pure, more preferably essentially pure. The compositions of this invention may contain a safe and effective amount of the retinoid, such that the resultant composition is safe and effective for regulating keratinous tissue condition, preferably for regulating visible and/or tactile discontinuities in skin, more preferably for regulating signs of skin aging, even more preferably for regulating visible and/or tactile discontinuities in skin texture associated with skin aging. The compositions preferably contain from or about 0.005 % to or about 2 %, more preferably 0.01 % to or about 2 %, retinoid. Retinol is preferably used in an amount of from or about 0.01 % to or about 0.15 %; retinol esters are preferably used in an amount of from or about 0.01 % to or about 2 % (e.g., about 1 %); retinoic acids are preferably used in an amount of from or about 0.01 % to or about 0.25 %; tocopheryl-retinoate, adapalene, and tazarotene are preferably used in an amount of from or about 0.01 % to or about 2 %.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01 % to 2 % by weight of the composition.

Compositions of the present invention may include vitamins. Illustrative vitamins are Vitamin A (retinol), Vitamin B₂, Vitamin B₃ (niacinamide), Vitamin B₆, Vitamin B12, Vitamin C, Vitamin D, Vitamin E, Vitamin K and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. A particularly suitable Vitamin B₆ derivative is Pyridoxine Palmitate. Flavonoids may also be useful, particularly glucosyl hesperidin, rutin, and soy isoflavones (including genistein, daidzein, equol, and their glucosyl derivatives) and mixtures thereof. Total amount of vitamins or flavonoids when present may range from 0.0001 to 10 %, preferably from 0.01 % to 1 %, optimally from 0.1 to 0.5 % by weight of the composition.

Another type of useful substance can be that of an enzyme such as oxidases, proteases, lipases and combinations. Particularly preferred is superoxide dismutase, commercially available as Biocell SOD from the Brooks Company, USA.

Desquamation promoters may be present. Illustrative are the monocarboxylic acids. Monocarboxylic acids may be substituted or unsubstituted with a carbon chain length of up to 16. Particularly preferred carboxylic acids are the alpha-hydroxycarboxylic acids, beta-hydroxycarboxylic or polyhydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic, lactic malic and tartaric acids. A representative salt that is particularly preferred is ammonium lactate. Salicylic acid is representative of the beta-hydroxycarboxylic acids. Amounts of these materials when present may range from about 0.01 to about 15 % by weight of the composition. Other phenolic acids include ferulic acid, salicylic acid, kojic acid and their salts.

A variety of herbal extracts may optionally be included in compositions of this invention. Illustrative are pomegranate, white birch (Betula Alba), green tea, chamomile, licorice and extract combinations thereof. The extracts may either be water soluble or water-insoluble carried in a solvent which respectively is hydrophilic or hydrophobic. Water and ethanol are the preferred extract solvents. Also included may be such materials as resveratrol, alpha-lipoic acid, ellagic acid, kinetin, retinoxytrimethylsilane (available from Clariant Corp. under the Silcare 1 M-75 trademark), dehydroepiandrosterone (DHEA) and combinations thereof. Ceramides (including Ceramide 1, Ceramide 3, Ceramide 3B, Ceramide 6 and Ceramide 7) as well as pseudoceramides may also be utilized for many compositions of the present invention but may also be excluded. Amounts of these materials may range from about 0.000001 to about 10 %, preferably from about 0.0001 to about 1 % by weight of the composition.

Colorants, opacifiers and abrasives may also be included in compositions of the present invention. Each of these substances may range from about 0.05 to about 5 %, preferably between 0.1 and 3 % by weight of the composition.

The compositions of the present invention may contain a safe and effective amount of a peptide active selected from pentapeptides, derivatives of pentapeptides, and mixtures thereof. As used herein, "pentapeptides" refers to both the naturally occurring pentapeptides and synthesized pentapeptides. Also useful herein are naturally occurring and commercially available compositions that contain pentapeptides. A preferred commercially available pentapeptide derivative-containing composition is Matrixyl™, which is commercially available from Sederma, France. The pentapeptides and/or pentapeptide derivatives are preferably included in amounts of from about 0.000001 % to about 10 %, more preferably from about 0.000001 % to about 0.1 %, even more preferably from about 0.00001 % to about 0.01 %, by weight of the composition. In embodiments wherein the pentapeptide-containing composition Matrixyl™ is used, the resulting composition preferably contains from about 0.01 % to about 50 %, more preferably from about 0.05 % to about 20 %, and even more preferably from about 0.1 % to about 10 %, by weight of the resulting composition of Matrixyl™.

Additional peptides, including but not limited to, di-, tri-, and tetrapeptides and derivatives thereof, and poly amino acid sequences of molecular weight from 200 - 20000. Amino acids may be naturally occurring or synthetic, dextro or levo, straight chain or cyclized and may be included in the compositions of the present invention in amounts that are safe and effective. As used herein, "peptides" refers to both the naturally occurring peptides and synthesized peptides. Also useful herein are naturally occurring and commercially available compositions that contain peptides.

Suitable dipeptides for use herein include Carnosine. Preferred tripeptides and derivatives thereof may be purchased as Biopeptide CL™. and a copper derivative sold commercially as lamin, from Sigma (St.Louis, Mo.).

Further ingredients useful in skin care compositions herein may be selected from any and all: skin conditioning agents, skin feel mildness agents, suspending agents, auxiliary thickening agents, viscosity control agents, dispersants, solubilizing/clarifying agents, stabilizers, opacifiers/pearlescent agents, chelating/sequestering agents, hydrotropes, bactericides/fungicides, antioxidants, pH control agents, buffering agents, colorants and perfumes/fragrances, water, other optional ingredients (auxiliary agents) and the like.

The compositions of the present invention can also be optionally, incorporated into a water insoluble substrate for application to the skin such as in the form of a treated wipe.

### Method of Making Compositions

Compositions within the scope of this invention were prepared in the following manner. Mix all water soluble ingredients including preservatives, thickening polymer, optionally glycerine, and water and heat to a temperature of 70-90°C. In a separate vessel mix all oil soluble ingredients including sugar surfactant and 12HSA to a temperature of 70-90°C. Add the oil phase to the water phase at a temperature of 70-90°C with agitation. Optionally add niacinamide at 45°C followed by addition of fragrance and phenoxyethanol at 40°C. Cool the mixture to room temperature with mixing.

### Method of Using Compositions

The composition according to the invention is intended primarily as a product for topical application to human skin, especially as an agent for conditioning and smoothening the skin, and preventing or reducing the appearance of wrinkled or aged skin, or age spots, or lightening of the skin.

In use, a small quantity of the composition, for example from 1 to 5 ml, is applied to exposed area of the skin, from a suitable container or applicator and, if necessary, it is then spread over and/or rubbed into the skin using the hand or fingers or a suitable device.

While the above summarizes the present invention, it will become apparent to those skilled in the art that modifications, variations and alterations may be made without deviating from the scope and spirit of the present invention as described and claimed herein. The invention will now be further illustrated in the following non-limiting examples.

### EXAMPLE 1

### 12-[(12-hydroxyoctadecanoyl)oxy] octadecanoic acid was prepared as follows:

### Reagents & Analytical Methods

All reagents and solvents were obtained from commercial sources (Sigma-Aldrich, Alfa Aesar, EMD Chemicals) and used without further purification unless otherwise indicated. Reaction monitoring was performed using either thin layer chromatography (TLC) or gas chromatography (GC). TLC was performed using silica gel 60 F₂₅₄ plates (EMD Chemicals) and visualizing by UV (254nm), 4 % phosphomolybdic acid in ethanol, 4 % ninhydrin in ethanol and/or using an iodine chamber. GC was performed on a Hewlett Packard 5890 Series II Plus Gas Chromatograph equipped with a HP-1 crosslinked methyl silicone gum (25m X 0.2 mm X 0.33 um film thickness) column operated by GC Chemstation (HP, version A.06.01 [403], Hewlett Packard) software and the following parameters and gradient applied; injector temp 250°C, oven temp: 70°C (2min), ramp at 25°C/min up to 250°C, held at 250°C (11min). High performance liquid chromatography (HPLC) was performed using a Waters 2695 Separations Module equipped with a Waters 2996 Photodiode Array Detector and operated with Empower Pro software (Waters Corp.). Liquid chromatography/mass spectrometry (LC-MS) was performed using a Finnigan Mat LCQ Mass Spectrometer via direct infusion of samples (50ppm) in methanol and the total ion count monitored using electrospray ionization in the (-) mode (ESI - ). ¹H and ¹³C Nuclear magnetic resonance (NMR) spectroscopy was performed using a Eft-60 NMR Spectrometer (Anasazi instruments, Inc.) and processed using WinNuts software (Acorn NMR, Inc.). Purities were determined by GC, TLC and LC/MS.

### Synthetic Procedures

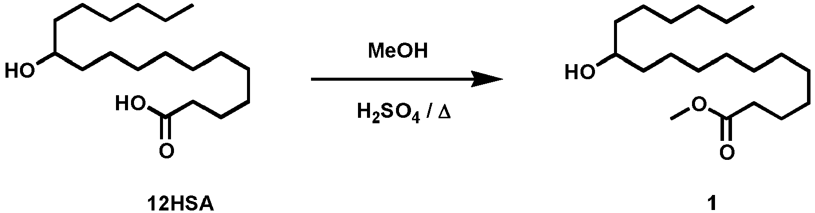

Methyl 12-hydroxyoctadecanoate (1) - Sulfuric acid (H₂SO₄) (7ml) was added to a solution of 12-hydroxyoctadecanoic acid (12HSA) (40g, 0.13mol) in methanol (MeOH) (700ml) and the resulting solution refluxed for 45min. At this time, TLC [50 µL aliquot into chloroform (CHCl₃) (500µL); eluted with 30 % ethyl acetate (EA) in hexanes (H)] showed the clean formation of a single product and no starting material (S.M.). Sodium bicarbonate (NaHCO₃) (12g) was added and the solvents removed under high vacuum (H.V.) at 50°C. The residue was diluted with t-butyl methyl ether (MTBE) (500ml) and washed successively with water:saturated NaHCO₃ (1:1, 500ml), saturated sodium chloride (NaCl) (500ml), dried with sodium sulfate (Na₂SO₄), filtered and the solvents removed under H.V. at 50°C to give product as a white solid (40.5g, 97 %).

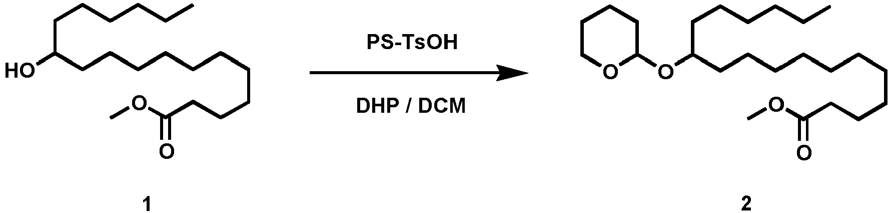

Methyl 12-(tetrahydro-2H-pyran-2-yloxy)octadecanoate (2) - Dihydropyran (DHP) (21.6ml, 0.254mol) was added to a solution of methyl 12-hydroxyoctadecanoate (1) (40g, 0.127mol) and polystyrene p-toluenesulfonic acid (PS-TsOH) (2.5mmol/g; 2.54g, 6.36mmol) in dichloromethane (DCM) (400ml) and the resulting mixture stirred at room temperature (R.T.) for 1.5hr. At this time, TLC [(40uL aliquot into MTBE:saturated NaHCO₃ (400µL:400µL); eluted with 5 % EA in H] showed the clean formation of a single product and no S.M. The solids were filtered off and the solution diluted with MTBE (1 L) and successively washed with saturated NaHCO₃ (250ml), saturated NaCl (250ml), dried with Na₂SO₄, filtered and the solvents removed under H.V. at 50°C to give product as an orange oil (55.8g, 110 %).

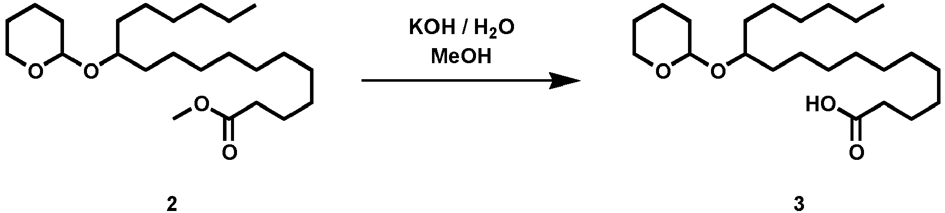

12-(tetrahydro-2H-pyran-2-yloxy)octadecanoic acid (3) - A solution of potassium hydroxide (KOH) (83.8g, 1.27mol) in MeOH (900ml) was added to methyl 12-(tetrahydro-2H-pyran-2-yloxy)octadecanoate (2) (50.6g, 0.127mol), followed by slow addition of water (60ml) and the solution stirred at room temperature (R.T.) for 1.5hr. At this time, TLC [(40uL aliquot into MTBE:1N HCl (400 µL:400 µL); eluted with 30 % EA in H] showed the clean formation of a single product and no S.M. The solvents were removed under H.V. at 45°C and the residue dissolved in water (200ml). The solution was cooled down in an ice bath and ice cold MTBE (1L) added, followed by addition of 2N HCl (700mL). The organic layer was separated and washed with saturated NaCl (200ml), dried with Na₂SO₄, filtered and the solvents removed under H.V. at below 10°C. Further drying under H.V. at <30°C afforded product as a light yellow paste (53.4g, 109 %).

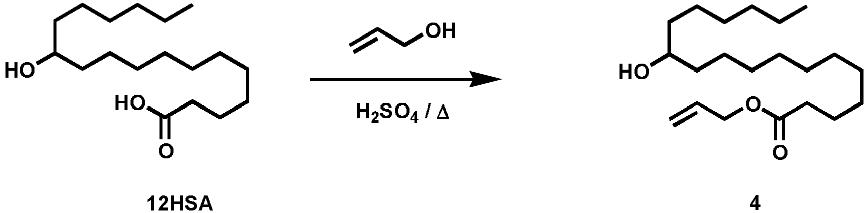

Allyl 12-hydroxyoctadecanoate (4) - Sulfuric acid (H₂SO₄) (5ml) was added to a solution of 12-hydroxyoctadecanoic acid (12HSA) (30g, 0.1mol) in allyl alcohol (AIIOH) (500ml) and the resulting solution refluxed for 1 hr. At this time, TLC [50µL aliquot into chloroform (CHCl₃) (500µL); eluted with 30 % EA in H] showed the clean formation of a single product and no S.M. Sodium bicarbonate (8.6g) was added and the solvents removed under H.V. at 50°C. The residue was diluted with t-butyl methyl ether (MTBE) (500ml) and washed successively with water:saturated NaHCO₃ (1:1, 500ml), saturated NaCl (500ml), dried with Na₂SO₄, filtered and the solvents removed under H.V. at 50°C to give product as a light amber-colored solid (34.6g, 101 %).

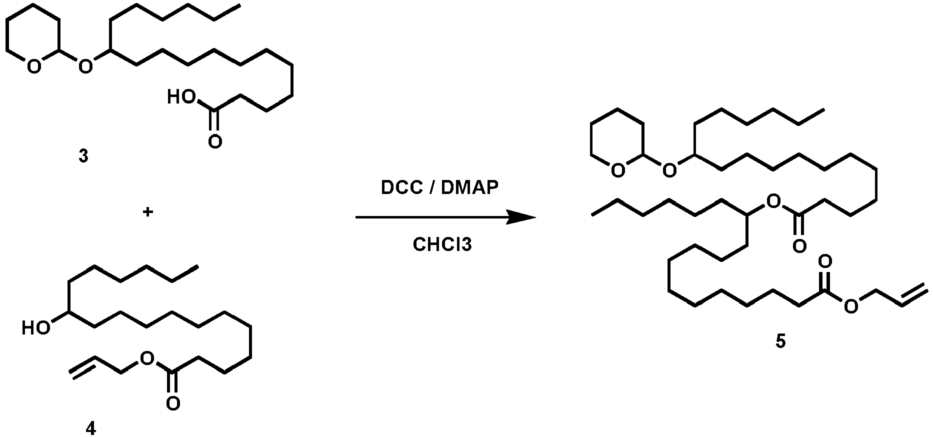

Allyl 12-([(12-(tetrahydro-2H-pyran-2-yloxy)octadecanoyl]oxy)octadecanoate (5)-Dicyclohexylcarbodiimide (DCC) (28.7g, 0.139mol) was added to a solution of 12-(tetrahydro-2H-pyran-2-yloxy)octadecanoic acid (3) (41.2g, 0.107mol) and allyl 12-hydroxyoctadecanoate (4) (34.6g, 0.102mol) in CHCl₃ (400ml), followed by N,N-dimethylaminopyridine (DMAP) (3.92g, 32.1mmol) and the resulting solution stirred at room temperature for 20hours. At this time, TLC [(40µL aliquot into MTBE:1N HCl (400µL:400µL); eluted with 10 % EA in H] showed the formation of a major product and small amounts of S.M. The solids were filtered off, washed with CHCl₃ (600ml) and the solvents removed under H.V. The residue was dissolved in MTBE (1L) and washed sequentially with saturated NaHCO₃ (1 X 800ml), 10mM HCl (1 X 800ml), saturated NaCl (1 X 800ml), dried with Na₂SO₄, filtered and the solvents removed under H.V. to give crude product as a yellow oil (81.3g). Further purification on silica gel eluting with 0-5 % EA in H afforded product as a colorless oil (45g, 66 %).

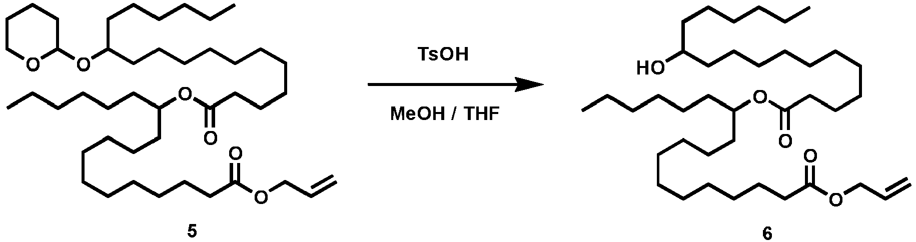

Allyl 12-[(12-hydroxyoctadecanoyl)oxy]octadecanoate (6) - p-Toluenesulfonic acid monohydrate (TsOH) (2.42g, 12.7mmol) was added to a solution of allyl 12-([(12-(tetrahydro-2H-pyran-2-yloxy)octadecanoyl]oxy)octadecanoate (5) (45g, 63.6mmol) in tetrahydrofuran (THF):MeOH (1:1; 540ml) and the resulting solution stirred at 40°C for 1hr. At this time, TLC [(10µL aliquot into MTBE: saturated NaHCO₃ (200µL:200µL); eluted with 10 % EA in H] showed the formation of two major products and no S.M. Potassium carbonate (K₂CO₃) (2M, 3.2ml) was added and the solvents removed under H.V. at 50°C. The residue was dissolved in MTBE (350ml) and successively washed with saturated NaHCO₃ (1 X 150ml), saturated NaCl (1 X 150ml), dried with Na₂SO₄, filtered and the solvents removed under H.V. to give crude product as a yellow oil (39.6g, 100 %).

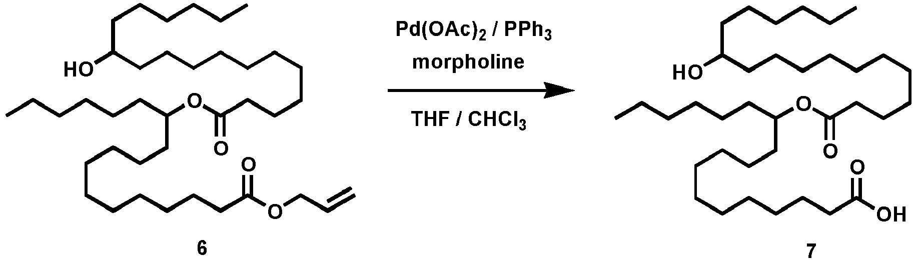

12-[(12-hydroxyoctadecanoyl)oxy]octadecanoic acid (7) - A solution of palladium (II) diacetate [Pd(OAc)₂] (1.40g, 6.26mmol) and triphenylphosphine (PPh₃) (6.57g, 25.0mmol) in CHCl₃ (350ml) was added to a solution of allyl 12-[(12-hydroxyoctadecanoyl)oxy]octadecanoate (6) (39g, 62.6mmol) and morpholine (54.5g, 626mmol) in THF (230ml) at RT and the solution stirred for 1 hr. At this time, TLC [(40uL into MTBE:0.1N HCl (400µL:400µL); eluted with 30 % EA in H] showed the formation of a major product. The solvents were removed under H.V. at 34°C and the residue diluted with MTBE (500ml) and successively washed with 1 N HCl (3 X 300ml), saturated NaCl (1 X 300ml), dried with Na₂SO₄, filtered and the solvents removed under H.V. to give an oil containing solids. The mixture was filtered through a plug of celite, washed with MTBE and the solvents stripped to give an orange oil (40.5g). The crude material was pre-purified by flash chromatography on silica gel eluting with 25-30 % EA in H. Further purification was achieved by suspending the sample in saturated NaHCO₃ (800ml), washing with hexane (3 x 800ml) after stirring the bi-phasic mixture vigorously, reacidifying with conc. HCl to pH 1 and extracting with MTBE (800ml). The organic layer was washed with saturated NaCl (500ml), dried with Na₂SO₄, filtered and the solvents removed under H.V. to give pure product as a thick colorless oil which solidifies upon standing at room temperature (10.7g, 29 %). Purity was shown to be >98 % by LC-MS and TLC. ¹H NMR (60 MHz, CDCl₃) □ 0.81 (t, 6H, terminal CH₃), 1.29 (m, 56H, carbon chain CH₂), 2.30 (m, 4H, R**CH₂**CO₂R), 3.61 (m, 1H, R**CH**OHR), 4.89 (m, 1 H, R**CH**(O₂CR)R), 6.57 (s, 2H, O**H** and CO₂**H**). LC-MS (ESI (-) m/z 582 (M-H⁺, 100 %).

### EXAMPLE 2

The effect of 12HSA and 12-[(12-hydroxyoctadecanoyl)oxy] octadecanoic acid on differentiation in primary human keratinocytes was evaluated.

The compounds were dissolved in DMSO at 10mM as the initial stock solutions and then diluted to appropriate final concentrations, 0.1µM, 1µM, 10µM for 12-[(12-hydroxyoctadecanoyl)oxy] octadecanoic acid and 1µM, 10µM for the monomer with culture medium for treatments. 0.1 % DMSO was used as the vehicle control. Primary human keratinocytes (Passage 2) grown to 70 % confluency in EpiLife medium (Gibco MEP1500CA, 0.06mM Calcium), trypsinized, cell number counted and Re-suspended in medium to make final cell counts at 5x10⁴/ml. 100, 000 cells seeded per well in a 6 well plate. The cells again grown to 70 % confluency, and then treated with 12-HSA monomer, 12-[(12-hydroxyoctadecanoyl)oxy] octadecanoic acid, and 0.1 % DMSO as control. Treated cells incubated for 24h and 48h, and harvested at the corresponding time points. Proteins were extracted from the cultures and protein concentration was quantified by Quick start Bradford dye reagent (BioRad #500-0205). 5ug protein samples were subjected to 4-20 % protein gel (BioRad Criterion #345-0034), separated by running gel at 175Volt for about 1 hour. Proteins were transferred to PVDF membrane (iblot Gel transfer stacks PVDF, Invitrogen IB4010-01). The PVDF membrane was blocked in 1 %BSA/PBS for 30 min and incubated with involucrin primary antibody (1:4000 dilution) in 1 %BSA/PBS at 4°C O/N (Sigma 19018), washed three times with TBS-Tween (Sigma T-9039) for 15 min each. The membrane then incubated with a secondary antibody in 1 %BSA/PBS at RT for 1 h (Cy5-conjugated Affipure Goat anti mouse IgG, Jackson ImmunoResearch Lab #115-175-003), washed three times with TBS-Tweenfor 15 min each, and scanned with Licor/Odyssey (Li-Cor Bioscience). The images obtained were analyzed for the involucrin bands for signal intensity by Odyssey v3.0 analysis program (Li-Cor Bioscience).

Involucrin expression data was presented in the following table.

**TABLE 1**

| Treatment | Involucrin expression (arbitrary unit) at day 1 | Treatment | Involucrin expression (arbitrary unit) at day 2 |
|---|---|---|---|
| 0.1 % DMSO | 27.85 | 0.1 % DMSO | 33.91 |
| 0.1µM12-[(12-hydroxyoctadecanoyl) oxy] octadecanoic acid | 35.85 | 0.1µM12-[(12-hydroxyoctadecanoyl) oxy] octadecanoic acid | 32.61 |
| 1µM12-[(12-hydroxyoctadecanoyl) oxy] octadecanoic acid | 36.67 | 1µM12-[(12-hydroxyoctadecanoyl) oxy] octadecanoic acid | 33.14 |
| 10µM12-[(12-hydroxyoctadecanoyl) oxy] octadecanoic acid | 33.77 | 10µM12-[(12-hydroxyoctadecanoyl)] oxy] octadecanoic acid | 29.46 |
| 1µM 12HSA | 26.7 | 1µM 12HSA | 56.03 |
| 10µM 12HSA | 34.51 | 10µM 12HSA | 69.52 |

The data clearly demonstrated that 12HSA stimulated involucrin expression compared to vehicle control (0.1 % DMSO) at day 2, indicating that 12HSA stimulated human keratinocyte differentiation. 12-[(12-hydroxyoctadecanoyl)oxy] octadecanoic acid did not have an effect on keratinocyte differentiation.

### EXAMPLE 3

The effect of 12HSA and 12-[(12-hydroxyoctadecanoyl)oxy] octadecanoic acid on ATP production by primary human fibroblasts was assayed.

The samples were prepared as in Example 2, except the final concentrations were at 0.1µM, 1µM, 10µM, 100µM for both compounds. Again 0.1 % DMSO in media is used as the vehicle control. Primary human neonatal fibroblasts grown in DMEM medium (high glucose supplemented with 2 mM Lglutamine, 10 % fetal bovine serum, antibiotic) to 70 % confluency, trypsinized, and re-suspended in medium. 7,500 cells seeded per well in the inner 60 wells of a 96-well plate in the same DMEM medium and grown for 3 days. The medium removed and the cells washed with serum-free DMEM, and then dosed with 200µl serum-free DMEM medium containing appropriate concentrations of the testing compounds. Each dosing was replicated in 4 wells. After 24 hours, test compound solution was removed and the cells were treated with a fresh solution of the same treatment. After another 24 hours, the ATP production of the cultures was assayed by CellTiter-Glo luminescent Cell viability assay (Promega #G7572) and luminescence readings measured. Average readings were calculated for every treatment. Results were summarized in the following table.

**TABLE 2**

| treatment | Luminescence reading for 12HSA treatment (average) | Statistical analysis by Student's t-Test-12HSA vs control (p) | Luminescence reading for 12-[(12-hydroxyoctadecanoyl)oxy] octadecanoic acid treatment (average) | Statistical analysis by Student's t-Test-12-[(12 hydroxyoctadecanoyl)oxy] octadecanoic acid vs control (p) |
|---|---|---|---|---|
| 100µM | 2151797 | 0.695836453 | 2794979 | 0.016648302 |
| 10µM | 2304763 | 0.813252493 | 2594799 | 0.095127857 |
| 1µM | 2398308 | 0.509793307 | 2550326 | 0.143436486 |
| 0.1µM | 2359722 | 0.629862144 | 2261382 | 0.944161273 |
| 0.1 % DMSO | 2245457 | | 2245457 | |

Statistical analysis was done by student t-Test with the assumption that the two samples had two tailed distribution with equal variance. Since the luminescence readings obtained by CellTiter-Glo was a determination of the number of viable cells in culture based on quantitation of the ATP present, the statistically significant improvement in luminescence readings in 100µM 12-[(12-hydroxyoctadecanoyl)oxy] octadecanoic acid treated culture demonstrated that the 12-[(12-hydroxyoctadecanoyl)oxy] octadecanoic acid stimulated fibroblast energy production, but 12HSA did not.

### EXAMPLE 4

Various mixtures were prepared with common lipohilic cosmetically acceptable carrier that are liquid at temperatures up to 40°C. Observation were recorded in table 3.

**TABLE 3**

| Samples | Observations |
|---|---|
| 10 % 12HSA / 90 % Isopropyl palmitate 10 % 12-[(12-hydroxyoctadecanoyl)oxy] octadecanoic acid / 90 % Isopropyl myristate | hard gel liquid |
| 10 % 12HSA / 90 % cyclomethicone-D5 10 % 12-[(12-hydroxyoctadecanoyl)oxy] octadecanoic acid / 90 % cyclomethicone-D5 | soft gel flowable liquid |
| 10 % 12HSA / 90 % Sunflower seed oil 10 % 12-[(12-hydroxyoctadecanoyl)oxy] octadecanoic acid / 90 % Sunflower seed oil | hard gel clear liquid |
| 10 % 12HSA / 90 % Light Mineral Oil 10 % 12-[(12-hydroxyoctadecanoyl)oxy] octadecanoic acid / 90 % Light Mineral Oil | hard gel flowable liquid |
| 10 % 12HSA / 90 % DC200-10cS 10 % 12-[(12-hydroxyoctadecanoyl)oxy] octadecanoic acid / 90 % DC200-10cS | paste flowable liquid |
| 10 % 12HSA / 90 % isostearyl alcohol 10 % 12-[(12-hydroxyoctadecanoyl)oxy] octadecanoic acid / 90 % isostearyl alcohol | gel clear liquid |
| 10 % 12HSA / 90 % oleic acid 10 % 12-[(12-hydroxyoctadecanoyl)oxy] octadecanoic acid / 90 % oleic acid | gel clear liquid |
| 10 % 12HSA / 90 % Tween40 10 % 12-[(12-hydroxyoctadecanoyl)oxy] octadecanoic acid / 90 % Tween40 | gel clear liquid |

It can be seen that mixtures of 12-[(12-hydroxyoctadecanoyl)oxy] octadecanoic acid with indicated carriers resulted in flowable, or even clear, liquids, whereas 12HSA gelled the carrier.

### EXAMPLE 5

Herein is illustrated a lotion according to the present invention with a formula as outlined in Table 4 below. This formula is packaged in a standard polypropylene bottle with screw-top. A label around the outside of the bottle specifies that the composition has effectiveness against the signs of aging including removal of fine lines and wrinkles.

**TABLE 4**

| INGREDIENT | WEIGHT % |
|---|---|
| PHASE A | |
| Water | Balance |
| Disodium EDTA | 0.05 |
| Methyl Paraben | 0.15 |
| Magnesium Aluminum Silicate | 0.60 |
| Triethanolamine | 1.20 |
| 12-[(12-hydroxyoctadecanoyl)oxy] octadecanoic acid | 0.0001 |
| PHASE B | |
| Xanthan Gum | 0.20 |
| Natrosol® 250HHR (ethyl cellulose) | 0.50 |
| Butylene Glycol | 3.00 |
| Glycerin | 2.00 |
| PHASE C | |
| Sodium Stearoyl Lactylate | 0.10 |
| Glycerol Monostearate | 1.50 |
| Stearyl Alcohol | 1.50 |
| Isostearyl Palmitate | 3.00 |
| Silicone Fluid | 1.00 |
| Cholesterol | 0.25 |
| Sorbitan Stearate | 1.00 |
| Butylated Hydroxy Toluene | 0.05 |
| Vitamin E Acetate | 0.01 |
| PEG-100 Stearate | 2.00 |
| Stearic Acid | 3.00 |
| Propyl Paraben | 0.10 |
| Parsol MCX® | 2.00 |
| Caprylic/Capric Triglyceride | 0.50 |
| Hydroxycaprylic Acid | 0.01 |
| C12-15 Alkyl Octanoate | 3.00 |
| PHASE D | |
| Vitamin A Palmitate | 0.10 |
| Bisabolol | 0.01 |
| Vitamin A Acetate | 0.01 |
| Fragrance | 0.03 |
| Retinol 50C | 0.02 |
| Conjugated Linoleic Acid | 0.50 |

### EXAMPLE 7

A water-in-oil topical liquid make-up foundation according to invention is described in Table 5 below. This foundation is delivered via a glass screw-top capped bottle. The bottle is placed within an outer carton. Inside the carton is placed instructions for use including applying the foundation to the face to achieve improvements in the signs of aging including enhanced radiance.

**TABLE 6**

| INGREDIENT | WEIGHT % |
|---|---|
| PHASE A | |
| Cyclomethicone | 9.25 |
| Oleyl Oleate | 2.00 |
| Dimethicone Copolyol | 20.00 |
| PHASE B | |
| Talc | 3.38 |
| Pigment (Iron Oxides) | 10.51 |
| Spheron L-1500 (Silica) | 0.50 |
| PHASE C | |
| Synthetic Wax Durachem 0602 | 0.10 |
| Arachidyl Behenate | 0.30 |
| PHASE D | |
| Cyclomethicone | 1.00 |
| Trihydroxystearin | 0.30 |
| PHASE E | |
| Laureth-7 | 0.50 |
| Propyl Paraben | 0.25 |
| PHASE F | |
| Fragrance | 0.05 |
| PHASE G | |
| Water | balance |
| 12-[(12-hydroxyoctadecanoyl)oxy] | 0.0001 - 0.5 % |
| octadecanoic acid | |
| 12HSA | 1-3 % |
| Methyl Paraben | 0.12 |
| Propylene Glycol | 8.00 |
| Niacinamide | 4.00 |
| Glycerin | 3.00 |
| Sodium Chloride | 2.00 |
| Sodium Dehydroacetate | 0.30 |

### EXAMPLE 8

Illustrated herein is a skin cream incorporating a 12-[(12-hydroxyoctadecanoyl)oxy] octadecanoic acid. The cream is deposited in a wide-mouth jar with screw-cap top. Printed on the label of the jar are instructions that the cream will control the signs of aging such as hyperpigmentation and sagging skin.

**TABLE 7**

| INGREDIENT | WEIGHT % |
|---|---|
| Glycerin | 6.93 |
| Niacinamide | 5.00 |
| 12-[(12-hydroxyoctadecanoyl)oxy] octadecanoic acid | 0.1 |
| Permethyl 101A¹ | 3.00 |
| Sepigel 305² | 2.50 |
| Q2-1403³ | 2.00 |
| Linseed Oil | 1.33 |
| Arlatone 2121⁴ | 1.00 |
| Cetyl Alcohol CO-1695 | 0.72 |
| SEFA Cottonate⁵ | 0.67 |
| Tocopherol Acetate | 0.50 |
| Panthenol | 0.50 |
| Stearyl Alcohol | 0.48 |
| Titanium Dioxide | 0.40 |
| Disodium EDTA | 0.10 |
| Glydant Plus⁶ | 0.10 |
| PEG-100 Stearate | 0.10 |
| Stearic Acid | 0.10 |
| Purified Water | Balance |

| | |
|---|---|
| ¹ Isohexadecane, Presperse Inc., South Plainfield, NJ ² Polyacrylamide(and)C13-14 Isoparaffin(and) Laureth-7, Seppic Corporation, Fairfield, NJ ³ dimethicone(and)dimethiconol, Dow Corning Corp. Midland, MI ⁴ Sorbitan Monostearate and Sucrococoate, ICI Americas Inc., Wilmington, DE ⁵ Sucrose ester of fatty acid ⁶ DMDM Hydantoin (and) lodopropynyl Butylcarbamate, Lonza Inc., Fairlawn, NJ | |

### EXAMPLE 9

Illustrative of another cosmetic personal care composition incorporating 12-[(12-hydroxyoctadecanoyl)oxy] octadecanoic acid is the formula of Table 6. This composition is packaged in a plastic polypropylene tube with flexible side walls for pressing the composition through a tube orifice. Instructions are printed on the outside of the tube directing that the composition be applied to the face and that in a period from about 2 weeks to about 6 months, the signs of aging will have diminished.

**TABLE 8**

| INGREDIENT | WEIGHT % |
|---|---|
| Polysilicone-11 | 29 |
| Cyclomethicone | 59 |
| Petrolatum | 11 |
| 12-[(12-hydroxyoctadecanoyl)oxy] octadecanoic acid | 0.2 |
| Dimethicone Copolyol | 0.5 |
| Sunflowerseed Oil | 0.3 |

### Example 10

A skin conditioning lotion is prepared as follows:

| | |
|---|---|
| Ingredient | % by Weight |
| Water | Balance |
| Carbopol Ultrez 10 | 0.8 |
| Polyoxyethylene 21 stearyl ether | 0.4 |
| Polysorbate 60 | 0.3 |
| Glycerine | 10.0 |

| | |
|---|---|
| Preservative | 0.7 |
| Dimethicone crosspolymer | 10 |
| NaOH (50 %) | 0.5 |
| Dimethicone | 11.0 |
| 12-[(12-hydroxyoctadecanoyl)oxy] octadecanoic acid | 0.5 |
| Mineral oil | 2.0 |
| Polyethylene | 4 |
| Fragrance | 0.3 |

While described in terms of the presently preferred embodiments, it is to be understood that such disclosure is not to be interpreted as limiting. Various modifications and alterations will no doubt occur to one skilled in the art after having read the above disclosure.

## Claims

1. A leave-on non-solid skin conditioning composition comprising:
(a) from 0.0001 % to 10 % of 12-[(12-hydroxyoctadecanoyl)oxy] octadecanoic acid; and
(b) a cosmetically acceptable carrier;
wherein the non-solid skin conditioning composition is an emulsion or a cream.

2. A composition of claim 1 wherein the cosmetically acceptable carrier is lipophilic and is liquid at temperature up to 40°C.

3. A composition of claim 2 wherein mixtures of 12-[(12-hydroxyoctadecanoyl) oxy] octadecanoic acid and the cosmetically acceptable carrier are flowable liquid.

4. A composition of any one of the preceding claims wherein the composition further comprises 12-hydroxystearic acid.

5. A composition of any one of the preceding claims wherein the composition further comprises niacinamide.

6. A composition of any one of the preceding claims wherein the composition further comprises disodium fumarate.

7. A method of conditioning skin, the method comprising applying to the skin the composition of any one of the preceding claims.

## Patentansprüche

1. Auf der Haut verbleibende nicht-feste Hautpflegezusammensetzung, umfassend:
(a) 0,0001% bis 10% 12-[(12-Hydroxyoctadecanoyl)oxy]octadecansäue und
(b) einen kosmetisch annehmbaren Träger,
worin das nicht-feste Hautpflegemittel eine Emulsion oder eine Creme darstellt.

2. Zusammensetzung nach Anspruch 1, wobei der kosmetisch annehmbare Träger lipophil und bei einer Temperatur bis zu 40°C flüssig ist.

3. Zusammensetzung nach Anspruch 2, wobei Mischungen der 12-[(12-Hydroxyoctadecanoyl)oxy]octadecansäure und des kosmetisch annehmbaren Trägers eine fließfähige Flüssigkeit sind.

4. Zusammensetzung nach irgendeinem der vorhergehenden Ansprüche, wobei die Zusammensetzung des Weiteren 12-Hydroxystearinsäure umfasst.

5. Zusammensetzung nach irgendeinem der vorhergehenden Ansprüche, wobei die Zusammensetzung des Weiteren Niacinamid umfasst.

6. Zusammensetzung nach irgendeinem der vorhergehenden Ansprüche, wobei die Zusammensetzung des Weiteren Dinatriumfumarat umfasst.

7. Verfahren zur Hautpflege, wobei das Verfahren das Auftragen der Zusammensetzung nach irgendeinem der vorhergehenden Ansprüche auf die Haut umfasst.

## Revendications

1. Composition de conditionnement de la peau non solide sans rinçage comprenant :
(a) de 0,0001 à 10 % d'acide 12-[(12-hydroxy-octadécanoyl)oxy]octadécanoïque ; et
(b) un véhicule acceptable sur le plan cosmétique ;
dans laquelle la composition de conditionnement de la peau non solide est une émulsion ou une crème.

2. Composition selon la revendication 1 dans laquelle le véhicule acceptable sur le plan cosmétique est lipophile et est liquide jusqu'à une température de 40 °C.

3. Composition selon la revendication 2 dans laquelle les mélanges d'acide 12-[(12-hydroxyoctadécanoyl)oxy]-octadécanoïque et de véhicule acceptable sur le plan cosmétique sont des liquides doués d'écoulement.

4. Composition selon l'une quelconque des revendications précédentes dans laquelle la composition comprend en outre de l'acide 12-hydroxystéarique.

5. Composition selon l'une quelconque des revendications précédentes dans laquelle la composition comprend en outre un niacinamide.

6. Composition selon l'une quelconque des revendications précédentes dans laquelle la composition comprend en outre du fumarate disodique.

7. Procédé de conditionnement de la peau, le procédé comprenant l'application à la peau de la composition selon l'une quelconque des revendications précédentes.
